(19) Europäisches Patentamt
European Patent Office
Office européen des brevets

(11) Veröffentlichungsnummer : **0 024 019**
**B1**

(12) # EUROPÄISCHE PATENTSCHRIFT

(45) Veröffentlichungstag der Patentschrift : **11.08.82**

(51) Int. Cl.$^3$ : **C 07 C 43/29**, C 07 C 41/16

(21) Anmeldenummer : **80104527.9**

(22) Anmeldetag : **31.07.80**

(54) **Verfahren zur Herstellung von 4-Fluor-3-phenoxy-toluol.**

(30) Priorität : **08.08.79 DE 2932093**

(43) Veröffentlichungstag der Anmeldung : **18.02.81 (Patentblatt 81/07)**

(45) Bekanntmachung des Hinweises auf die Patenterteilung : **11.08.82 Patentblatt 82/32**

(84) Benannte Vertragsstaaten : **BE CH DE FR GB IT LI NL**

(56) Entgegenhaltungen :
**DE A 2 242 519**
**DE A 2 745 006**
**DE B 2 304 006**

(73) Patentinhaber : **BAYER AG**
**Zentralbereich Patente, Marken und Lizenzen**
**D-5090 Leverkusen 1, Bayerwerk (DE)**

(72) Erfinder : **Fuchs, Rainer, Dr.**
**Roeberstrasse 8**
**D-5600 Wuppertal 1 (DE)**
Erfinder : **Maurer, Fritz, Dr.**
**Roeberstrasse 8**
**D-5600 Wuppertal 1 (DE)**
Erfinder : **Priesnitz, Uwe, Dr.**
**Heinrich Heine Strasse 42**
**D-4750 Unna-Massen (DE)**
Erfinder : **Riebel, Hans-Jochem, Dr.**
**In der Beek 92**
**D-5600 Wuppertal 1 (DE)**

**EP 0 024 019 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von 4-Fluor-3-phenoxy-toluol

Die Erfindung betrifft ein neues Verfahren zur Herstellung von 4-Fluor-3-phenoxy-toluol.

Es ist bekannt, daß bei der Reaktion von Fluor-benzolderivaten, welche neben Fluor weitere Halogensubstituenten, wie z.B. Chlor oder Brom, enthalten, mit Alkoholaten oder Phenolaten Fluor eher als andere Halogene substituiert wird.

So entsteht z.B. aus 4-Fluor-chlorbenzol und Kaliumphenolat 4-Chlor-diphenylether als Hauptprodukt (vergleiche DE-OS 2 619 489). Die Reaktion von Chlorpentafluorbenzol mit Natriumpentafluorphenolat führt zu einem Isomerengemisch aus 4-Chlor-nonafluor-diphenylether und 2-Chlor-nonafluor-diphenylether (vergleiche US-PS 3637866). Brom-2,3,4,6-tetrafluorbenzol reagiert mit Natriummethylat zu einem Isomerengemisch von Brom-trifluor-methoxy-benzolen (vergleiche J. Chem. Soc. Perkin II *1978*, 137-141).

Es ist weiter bekannt, daß Diarylether aus Halogenaromaten und Hydroxyaromaten in Gegenwart von Kupfer oder Kupferverbindungen als Katalysatoren hergestellt werden können, wobei jedoch kupferkatalysierte Enthalogenierung in jedem Fall als Nebenreaktion zu beobachten ist (vergleiche J. Chem. Soc. *1965*, 4953).

Es wurde nun gefunden, daß man 4-Fluor-3-phenoxy-toluol der Formel I

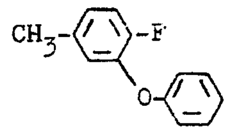

(I)

in guten Ausbeuten und hoher Reinheit erhält, wenn man 3-Brom-4-fluor-toluol der Formel II

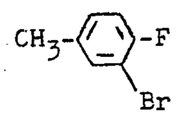

(II)

mit einem Alkali- oder Erdalkaliphenolat gegebenenfalls in Gegenwart eines Hilfsstoffes aus der Reihe der Alkali- und Erdalkalicarbonate und in Gegenwart von Kupfer oder einer Kupferverbindung als Katalysator sowie in Gegenwart von Isochinolin als Verdünnungsmittel bei Temperaturen zwischen 100 und 200 °C umsetzt.

Es ist als überraschend anzusehen, daß nach dem erfindungsgemäßen Verfahren praktisch nur 4-Fluor-3-phenoxytoluol aus 3-Brom-4-fluor-toluol entsteht, da nach dem Stand der Technik eher eine Substitution des Fluors unter Bildung von 3-Brom-4-phenoxy-toluol zu erwarten war. Ebenso ist es überraschend, daß eine Entbromierung zu 4-Fluor-toluol in weit geringerem Maße als bei der Durchführung der Reaktion nach literaturbekannten Methoden eintritt.

Die erfindungsgemäße Reaktion kann durch folgendes Formelschema skizziert werden :

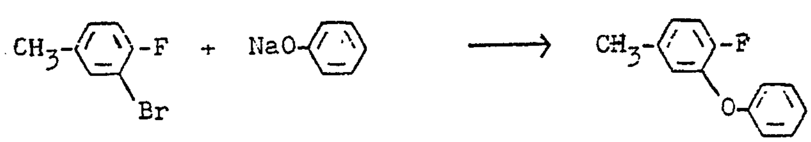

Das als Ausgangsverbindung zu verwendende 3-Brom-4-fluor-toluol ist bekannt (vergleiche Canad. Journ. Chem. *38* (1960), 2441-2449).

Alkali- oder Erdalkaliphenolate, welche als Ausgangsstoffe verwendet werden können, sind z.B. Natrium-, Kalium- oder Magnesiumphenolat. Natriumphenolat wird als Ausgangsverbindung bevorzugt. Hilfsstoffe aus der Reihe der Alkali- und Erdalkali-carbonate sind z.B. Kalium- und Magnesiumcarbonat. Diese Hilfsstoffe werden vorzugsweise dann verwendet, wenn als Ausgangsverbindung Natriumphenolat eingesetzt wird.

Als Katalysatoren werden Kupfer oder Kupferverbindungen verschiedener Oxidationsstufen verwendet. Beispielsweise seien Kupfer, Kupfer(I)oxid, Kupfer(I)chlorid und Kupfer(I)bromid genannt. Vorzugsweise wird Kupfer(I)oxid verwendet.

Die Reaktionstemperatur wird zwischen 100 und 200 °C, vorzugsweise zu Beginn der Umsetzung bei 100 bis 120 °C, dann bis Ende der Umsetzung bei 140 bis 180 °C gehalten.

Auf 1 Mol 3-Brom-4-fluor-toluol setzt man 1 bis 1,5 Mol, vorzugsweise 1 bis 1,2 Mol Phenolat, 0,75 bis 2 Mol, vorzugsweise 0,9 bis 1,5 Mol eines Hilfsstoffes aus der Reihe der Alkali- und Erdalkalicarbonate, 1 bis 50 g Kupfer-Katalysator und 150 bis 1 500 ml Isochinolin ein.

In einer bevorzugten Ausführungsform des erfindungsgemäßen Verfahrens wird 3-Brom-4-fluor-toluol zusammen mit dem Kupfer-Katalysator in Isochinolin vorgelegt und diese Mischung auf 100 bis 120 °C erhitzt. Dann gibt man dazu den Hilfsstoff und das Phenolat und erhöht die Innentemperatur auf etwa 160 °C. Bei dieser Temperatur wird das Reaktionsgemisch bis zum Reaktionsende gerührt. Die Aufarbeitung kann auf übliche Weise durchgeführt werden. Beispielsweise kann man nach Abkühlen und Filtrieren das Filtrat in einem mit Wasser nicht mischbaren Lösungsmittel, wie z.B. Cyclohexan, aufnehmen, die Lösung mit Salzsäure und Wasser waschen, trocknen, filtrieren und das Lösungsmittel abdestillieren. Das zurückbleibende Rohprodukt kann durch Vakuumdestillation gereinigt werden. Zur Charakterisierung dient der Siedepunkt.

4-Fluor-3-phenoxy-toluol kann durch Umsetzung mit N-Brom-succinimid in Gegenwart eines Radikalstarters, wie z.B. Azodiisobutyronitril, gegebenenfalls unter Verwendung eines Verdünnungsmittels, wie z.B. Tetrachlorkohlenstoff, bei Temperaturen zwischen 50 und 100 °C in 4-Fluor-3-phenoxy-benzylbromid umgewandelt werden. Diese Verbindung ist als Zwischenprodukt für Pestizide bekannt (vergleiche DE-OS 2 709 264).

## Herstellungsbeispiele

### Beispiel 1

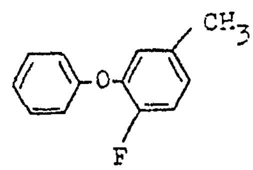

Zu einer Suspension von 189 g (1 Mol) 3-Brom-4-fluor-toluol und 50 g Kupfer(I)oxid in 250 ml Isochinolin wird bei 110 °C ein Gemisch von 84,3 g Magnesiumcarbonat (4 $MgCO_3 \cdot Mg(OH)_2 \cdot 4H_2O$) und 128 g (1,1 Mol) Natriumphenolat gegeben. Dann wird die Reaktionstemperatur auf 160 °C gesteigert, und es wird 15 Stunden bei dieser Temperatur nachgerührt. Danach wird abgekühlt und das Reaktionsgemisch wird filtriert. Das Filtrat wird in Cyclohexan aufgenommen. Die Cyclohexan-Lösung wird mit Salzsäure und Wasser gewaschen, über Natriumsulfat getrocknet und dann eingeengt. Nach dem Destillieren erhält man 170 g (84,1 % der Theorie) 4-Fluor-3-phenoxy-toluol in Form eines hellgelben Öls vom Siedepunkt 105 °C/ 6 mbar.

### Beispiel 2

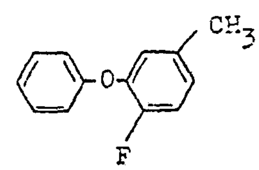

Verwendet man an Stelle von Magnesiumcarbonat 139 g (1 Mol) Kaliumcarbonat, so erhält man nach der gleichen Methode wie unter Beispiel 1 beschrieben 140 g (70 % der Theorie) 4-Fluor-3-phenoxy-toluol.

### Beispiel 3

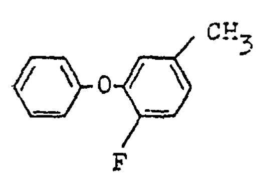

Verfährt man wie in Beispiel 1 und arbeitet das Reaktionsgemisch durch wiederholte fraktionierte Destillation auf, erhält man 141 g (70 % der Theorie) in Form eines hellgelben Öls vom Siedepunkt 105 °C/6 mbar.

**Anspruch**

Verfahren zur Herstellung von 4-Fluor-3-phenoxy-toluol der Formel I

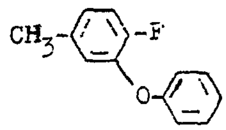

(I)

dadurch gekennzeichnet, daß man 3-Brom-4-fluor-toluol der Formel II

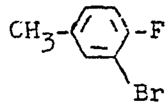

(II)

mit einem Alkali- oder Erdalkaliphenolat in Gegenwart von Kupfer oder einer Kupferverbindung als Katalysator, gegebenenfalls in Gegenwart eines Alkali- oder Erdalkalicarbonate sowie in Gegenwart von Isochinolin als Verdünnungsmittel bei Temperaturen zwischen 100 und 200 °C umsetzt.

**Claim**

Process for the preparation of 4-fluoro-3-phenoxy-toluene of the formula I

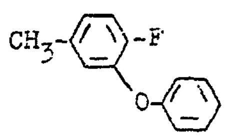

(I)

characterised in that 3-bromo-4-fluoro-toluene of the formula II

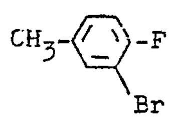

(II)

is reacted with an alkali metal phenolate or alkaline earth metal phenolate in the presence of copper or a copper compound as the catalyst, if appropriate in the presence of an alkali metal carbonate or alkaline earth metal carbonate, and in the presence of isoquinoline as the diluent, at temperatures between 100 and 200 °C.

**Revendication**

Procédé de fabrication de 4-fluoro-3-phénoxy-toluène de formule I

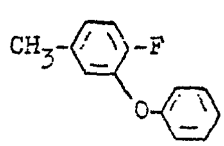

(I)

caractérisé en ce qu'on fait réagir du 3-bromo-4-fluoro-toluène de formule II

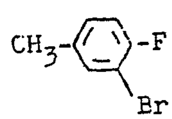

(II)

avec un phénolate alcalin ou alcalino-terreux en présence de cuivre ou d'un composé de cuivre comme catalyseur, éventuellement en présence d'un carbonate alcalin ou alcalino-terreux ainsi qu'en présence d'isoquinoléine comme diluant à des températures entre 100 et 200 °C.